(19) Europäisches Patentamt
European Patent Office
Office européen des brevets

(11) **EP 4 693 326 A2**

(12) **EUROPEAN PATENT APPLICATION**

(43) Date of publication:
**11.02.2026 Bulletin 2026/07**

(21) Application number: **25224576.6**

(22) Date of filing: **01.02.2021**

(51) International Patent Classification (IPC):
**G16H 20/17** (2018.01)

(52) Cooperative Patent Classification (CPC):
**G16H 20/17**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.02.2020 US 202016780278**

(62) Document number(s) of the earlier application(s) in accordance with Art. 76 EPC:
**21710641.8 / 4 100 959**

(71) Applicant: **Insulet Corporation**
**Acton, MA 01720 (US)**

(72) Inventors:
• **CARDINALI, Steven**
**Tewksbury, Massachusetts, 01876 (US)**
• **ZADE, Ashutosh**
**San Diego, California, 92128 (US)**
• **LEE, Joon Bok**
**Acton, Massachusetts, 01720 (US)**
• **O'CONNOR, Jason**
**Acton, Massachusetts, 01720 (US)**
• **ZHENG, Yibin**
**Hartland, Wisconsin, 53029 (US)**

(74) Representative: **Peterreins Schley**
**Patent- und Rechtsanwälte PartG mbB**
**Hermann-Sack-Straße 3**
**80331 München (DE)**

Remarks:
This application was filed on 17-12-2025 as a divisional application to the application mentioned under INID code 62.

(54) **ACCURATE PREDICTION OF QUANTITY AND TIMING OF INSULIN RESERVOIR REFILLS**

(57) Exemplary embodiments may provide an improved approach to automated insulin delivery by more accurately estimating the total daily insulin (TDI) of a user. As a result, less insulin is wasted by the delivery system, and the estimate of TDI more closely matches a user's actual daily insulin needs. Hence, the user need not refill the insulin reservoir excessively or need not fret unnecessarily about running out of insulin prematurely. The estimate relies on the history of actual automated insulin deliveries and thus reflects the actual insulin delivered to the user more accurately than conventional approaches.

Figure 6

**Description**

**CROSS-REFERENCE TO RELATED APPLICATIONS**

This application claims the benefit of the filing date of U.S. Non-Provisional Application Serial No. 16/780,278, filed February 3, 2020, the entire contents of which are incorporated herein by reference in their entirety.

**BACKGROUND**

Insulin held in insulin reservoirs of automated drug delivery devices is often wasted. This is due in part to excessive amounts of insulin being present in the insulin reservoirs. As a result, not all of the insulin in reservoir may be used before the insulin must be discarded. Typically, the insulin in such reservoirs is only suitable for use for a period of three days. There is a risk of infection at the injection site if the insulin is not discarded in a timely fashion. Given that insulin is expensive, this discarding of insulin is especially problematic.

Insulin reservoirs may be filled to standardized levels for all users. Unfortunately, this approach may result in insulin reservoirs containing excessive insulin that needs to be discarded for many users. Insulin reservoirs may be filled to levels that err on being excessive so as to not run out of insulin for a specified period. This problem can be especially acute for users that require less insulin than normal. In other instances, the amount of insulin filled in the insulin reservoir may be too little. This requires the reservoir to be filled frequently, which can result in unnecessary loss of insulin due to priming as part of the refilling.

**SUMMARY**

In accordance with an exemplary embodiment, a method is performed by a processor of an electronic device. The method includes estimating total daily basal insulin needs of a user based on a historic amount of insulin delivered by an automated insulin delivery system to the user per day. The total daily bolus insulin needs of the user also are estimated based on past insulin bolus deliveries to the user. The estimate of the total daily basal insulin needs of the user is added with the estimate of the total daily bolus insulin needs of the user to obtain a daily estimate of insulin needs for the user. A determination of how many days of insulin for the user are to be stored in a reservoir for the automated insulin delivery system is made. The amount of insulin to be filled in the reservoir is determined to be equal to the daily estimate of insulin needs for the user multiplied by the determined number of days of insulin for the user that are to be stored in the reservoir.

The estimating of the total daily insulin needs may include determining an average dosage of insulin delivered by the automated insulin delivery system for a period and multiplying the average dosage by the number of periods in a day to calculate the amount of insulin delivered by the automated insulin delivery system to the user per day. The estimating of the total daily insulin needs of the user may include multiplying the amount of insulin delivered by the automated insulin delivery system to the user per day by a factor, such as two. The estimate of the total daily bolus insulin needs of the user may be adjusted by a safety factor before adding with the estimate of the total daily basal insulin needs of the user. The estimating of the total daily bolus insulin needs of the user may include subtracting two times the estimated total daily basal insulin needs of a user from an average overall sum of insulin deliveries to the user, including basal deliveries and bolus deliveries. A prompt to the user may be generated via a user interface to have the determined amount of insulin to be filled added to the insulin reservoir. Instructions for practicing the method may be stored on a non-transitory computer-readable storage medium.

In accordance with an exemplary embodiment, an electronic device includes a storage for storing a program for determining an amount of insulin to be filled into an insulin reservoir of an insulin delivery system for a user, a history of automated insulin deliveries by the insulin delivery system to the user and a history of bolus insulin deliveries for the user. The electronic device also includes a processor for executing the program to estimate total daily basal insulin needs of a user based on a historical amount of insulin delivered by an automated insulin delivery system to the user per day. The processor also estimates total daily bolus insulin needs of the user based on past insulin bolus deliveries to the user. The processor adds the estimate of the total daily basal insulin needs of the user with the estimate of the total daily bolus insulin needs of the user to obtain a daily estimate of insulin needs for the user. The processor determines how many days of insulin for the user are to be stored in a reservoir for the automated insulin delivery system and determines that the amount of insulin to be filled in the reservoir is equal to the daily estimate of insulin needs for the user multiplied by the determined number of days of insulin for the user that are to be stored in the reservoir.

In accordance with an exemplary embodiment, a method is performed by a processor of an electronic device. Per the method, when insulin is added to an insulin reservoir of an insulin pump, a query as to how much insulin was added to the reservoir is made. In response to the querying, a response is received that specifies an amount of insulin that was added to the reservoir. A determination is made regarding when the insulin reservoir will need to be replaced or refilled based on the amount of insulin added to the insulin reservoir and historic data of a user's actual insulin use.

The determining of when the insulin reservoir will need to be replaced or refilled is updated based on additional insulin being added to the insulin reservoir and/or historic data of the user's actual insulin use being updated. The insulin reservoir may need to be replaced or refilled when an amount of insulin in the insulin reservoir is below a threshold. The insulin reservoir may need to be replaced or refilled when the insulin in the insulin reservoir has been in the reservoir past a time deadline. Instructions for practicing the method may be stored on a non-transitory computer-readable storage medium.

In accordance with an exemplary method, a method is performed by a processor of an electronic device. Per the method, an indication of a duration for which an amount of insulin in an insulin reservoir of an insulin pump is to be above a threshold amount is received. A determination is made of an amount of insulin to be added to the insulin reservoir or replaced in the insulin reservoir in order to keep the amount of insulin in the insulin reservoir above the threshold amount for the specified duration based on historic data of a user's actual insulin use. Prompting the determined amount of insulin to be added to the reservoir or to be replaced in the insulin reservoir. The determining of the amount of insulin to be added may include multiplying the average daily insulin use for the user by the duration specified in days. Instructions for practicing the method may be stored on a non-transitory computer-readable storage medium.

## BRIEF DESCRIPTION OF THE DRAWINGS

Figure 1 depicts a block diagram of a drug delivery system suitable for practicing exemplary embodiments.
Figure 2 depicts a block diagram showing options for replenishing an expired insulin reservoir.
Figure 3 depicts a diagram of types of devices suitable for practicing methods described herein.
Figure 4 depicts a block diagram of a device suitable for practicing methods described herein.
Figure 5 depicts a flowchart showing illustrative steps for determining actual insulin daily deliveries for a user.
Figure 6 depicts a flowchart showing illustrative steps for estimating an amount of insulin needed to fill the insulin reservoir for a user.
Figure 7 depicts a flowchart showing illustrative steps for determining an estimate of daily bolus needs for a user.
Figure 8 depicts a flowchart showing illustrative steps for determining an amount of time remaining before insulin in the insulin reservoir will be consumed based on average insulin needs of the user.

## DETAILED DESCRIPTION

Exemplary embodiments may provide an improved approach to automated insulin delivery by more accurately estimating the total daily insulin (TDI) of a user. As a result, less insulin is wasted by the delivery system, and the estimate of TDI more closely matches a user's actual daily insulin needs. Hence, the user need not refill the insulin reservoir excessively or need not fret unnecessarily about running out of insulin prematurely. The estimate relies on the history of actual automated insulin deliveries and thus reflects the actual insulin delivered to the user more accurately than conventional approaches.

Exemplary embodiments may use this more accurate estimate of TDI to determine how much insulin needs to be filled into an insulin reservoir. The TDI may be used to estimate daily basal insulin needs of a user. The estimate of daily basal insulin for the user may be added to an estimate of daily bolus insulin needs to obtain an estimate of total daily insulin needs for the user. The estimate of daily bolus needs is obtained from the actual bolus insulin delivery history of the user. Once the estimate of daily insulin needs for the user is determined, a determination of how many days of insulin are to be held in the reservoir is made. The total amount of insulin to be contained in the reservoir is then calculated by multiplying the number of days of insulin to be held in the reservoir by the estimate of daily insulin needs of the user. The reservoir may be filled with the calculated amount of insulin. This approach provides a more accurate estimate of the amount of insulin needed in the reservoir for the designated number of days than conventional approaches and avoids both needing to refill the reservoir prematurely or overfilling the reservoir so that costly insulin is wasted.

Exemplary embodiments also may provide an accurate estimate of the amount of time before a reservoir needs to be replenished. In these exemplary embodiments, the user may be asked to state the quantity of insulin that filled the reservoir at the time of filling the reservoir. Based on this quantity, the exemplary embodiments may estimate the time remaining before the insulin expires using the user's actual insulin delivery history rather than an estimate based solely on the basal insulin delivery amount or other clinical parameters that may be inaccurate. The time remaining may be determined based on the estimate of user's daily insulin needs discussed above.

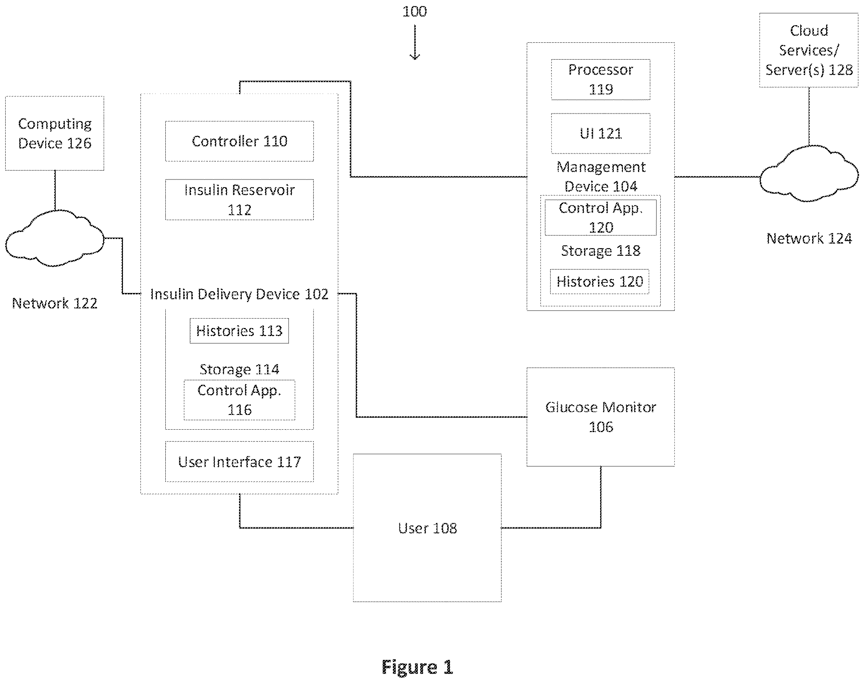

Figure 1 depicts an illustrative drug delivery system 100 that is suitable for delivering insulin to the user 108 in an exemplary embodiment. The drug delivery system 100 includes an insulin delivery device 102. The insulin delivery device 102 may be a wearable device that is worn on the body of the user 108. The insulin delivery device 102 may be directly coupled to a user (e.g., directly attached to a body part and/or skin of the user 108 via an adhesive or the like). In an example, a surface of the insulin delivery device 102 may include an adhesive to facilitate attachment to the user 108.

The insulin delivery device 102 may include a controller 110. The controller 110 may be implemented in hardware, software, or any combination thereof. The controller 110 may, for example, be a microprocessor, a logic circuit, a field programmable gate array (FPGA), an application specific integrated circuit (ASIC) or a microcontroller coupled to a memory. The controller 110 may maintain a date and time as well as other functions (e.g., calculations or the like). The controller 110 may be operable to execute a control application 116 stored in the storage 114 that enables the controller 110 to direct operation of the insulin delivery device 102. The storage 114 may hold histories 113 for a user, such as a history of automated insulin deliveries, a history of bolus insulin deliveries, meal event history, exercise event history and the like. In addition, the controller 110 may be operable to receive data or information. The storage 114 may include both primary memory and secondary memory. The storage may include random access memory (RAM), read only memory (ROM), optical storage, magnetic storage, removable storage media, solid state storage or the like.

The insulin delivery device 102 may include an insulin reservoir 112 for storing insulin for delivery to the user 108 as warranted. A fluid path to the user 108 may be provided, and the insulin delivery device 102 may expel the insulin from the insulin reservoir 112 to deliver the insulin to the user 108 via the fluid path. The fluid path may, for example, include tubing coupling the drug delivery device 102 to the user 108 (e.g., tubing coupling a cannula to the insulin reservoir 112).

There may be one or more communications links with one or more devices physically separated from the insulin delivery device 102 including, for example, a management device 104 of the user and/or a caregiver of the user and/or a glucose monitor 106. The communication links may include any wired or wireless communication link operating according to any known communications protocol or standard, such as Bluetooth®, Wi-Fi, a near-field communication standard, a cellular standard, or any other wireless protocol The insulin delivery device 102 may also include a user interface 117, such as an integrated display device for displaying information to the user 108 and in some embodiments, receiving information from the user 108. The user interface 117 may include a touchscreen and/or one or more input devices, such as buttons, knob or a keyboard.

The insulin delivery device 102 may interface with a network 122. The network 122 may include a local area network (LAN), a wide area network (WAN) or a combination therein. A computing device 126 may be interfaced with the network, and the computing device may communicate with the insulin delivery device 102. The role that the computing device 126 may play in the exemplary embodiments will be described in more detail below.

The drug delivery system 100 may include a glucose monitor 106 for sensing the blood glucose concentration levels of the user 108. The glucose monitor 106 may provide periodic blood glucose concentration measurements and may be a continuous glucose monitor (CGM), or another type of device or sensor that provides blood glucose measurements. The glucose monitor 106 may be physically separate from the insulin delivery device 102 or may be an integrated component thereof. The glucose monitor 106 may provide the controller 110 with data indicative of measured or detected blood glucose levels of the user 108. The glucose monitor 106 may be coupled to the user 108 by, for example, adhesive or the like and may provide information or data on one or more medical conditions and/or physical attributes of the user 108. The information or data provided by the glucose monitor 106 may be used to adjust drug delivery operations of the insulin delivery device 102.

The drug delivery system 100 may also include the management device 104. The management device 104 may be a special purpose device, such as a dedicated personal diabetes manager (PDM) device. The management device 104 may be a programmed general purpose device, such as any portable electronic device including, for example, a dedicated controller, such as processor, a smartphone, or a tablet. The management device 104 may be used to program or adjust operation of the drug delivery device 102 and/or the sensor 104. The management device 104 may be any portable electronic device including, for example, a dedicated controller, a smartphone, or a tablet. In the depicted example, the management device 104 may include a processor 119 and a storage 118. The processor 119 may execute processes to manage a user's blood glucose levels and for control the delivery of the drug or therapeutic agent to the user 108. The processor 119 may also be operable to execute programming code stored in the storage 118. For example, the storage may be operable to store one or more control applications 120 for execution by the processor 119. The storage 118 may store the control application 120, histories 120 like those described above for the insulin delivery device 102 and other data and/or programs.

The management device 104 may include a user interface 121 for communicating with the user 108. The user interface may include a display, such as a touchscreen, for displaying information. The touchscreen may also be used to receive input when it is a touch screen. The user interface 121 may also include input elements, such as a keyboard, button, knobs or the like.

The management device 104 may interface with a network124, such as a LAN or WAN or combination of such networks. The management device 104 may communicate over network 124 with one or more servers or cloud services 128. The role that the one or more servers or cloud services 128 may play in the exemplary embodiments will be described in more detail below.

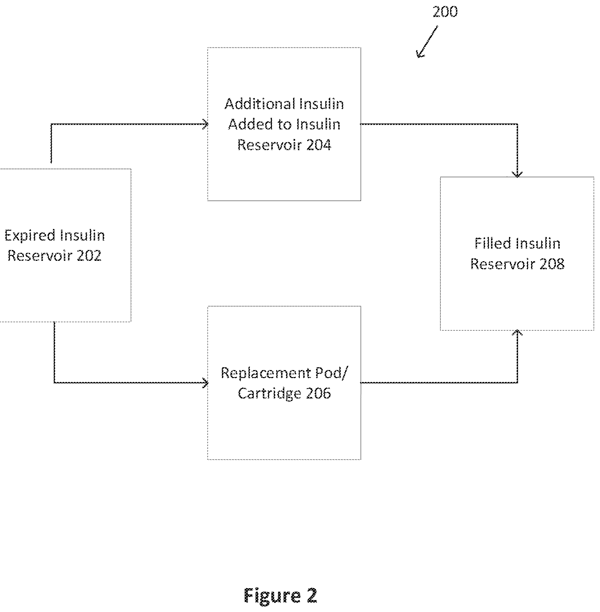

Figure 2 depicts a block diagram 200 of a situation encountered with insulin delivery systems. When the amount of insulin in an insulin reservoir gets too low, the insulin reservoir is deemed to have expired and needs filling or replacement. Thus, in Figure 2, expired reservoir 202 represents such an insulin reservoir that has expired. The expired reservoir 202 may be refillable, such that the insulin reservoir structure remains intact but additional insulin in added to the insulin reservoir 204. Another alternative is the that insulin is stored is a discardable pod or cartridge that is disposed of at the time of expiration. A replacement pod or cartridge 206 preloaded with insulin is added to the insulin delivery device 102. The result of both of these approaches is a filled insulin reservoir 208.

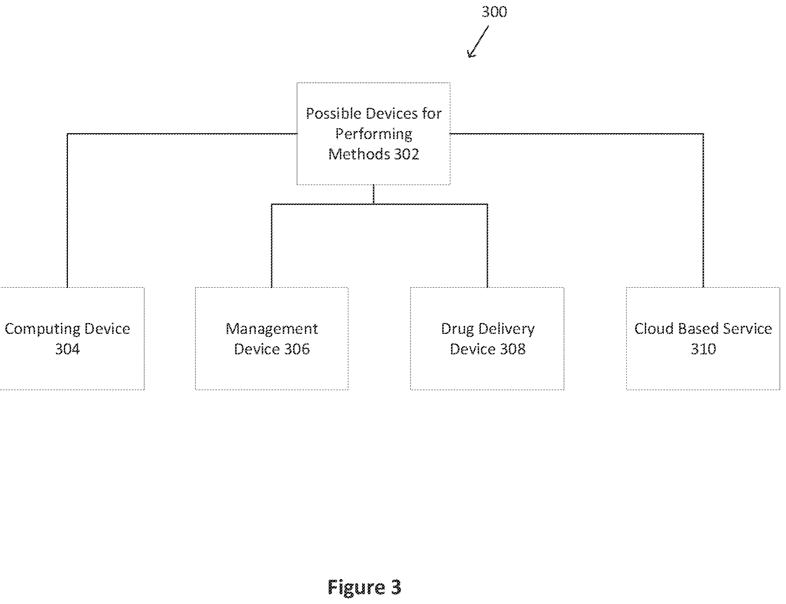

The methods described herein for the exemplary embodiments relating to determining an accurate estimate of a user's daily insulin needs, determining how much insulin to add to an insulin reservoir to fill the reservoir for a user and determining how long before an insulin reservoir will expire may be performed by a number of different types of devices as shown in the diagram 300 of Figure 3. Figure 3 depicts possible devices 302 for performing such methods. These methods may be performed by a computing device 304. The computing device may be, for example, computing device 126 that is interfaced with network 122 or a server 128 interfaced with network 124. The methods may also be performed by a management device 306, such as management device 104. Further, the methods may be performed by a drug delivery device 308, such as insulin delivery device 102. Still further, the methods may be performed by a cloud based service 310 like cloud based services 128 accessible on network 124.

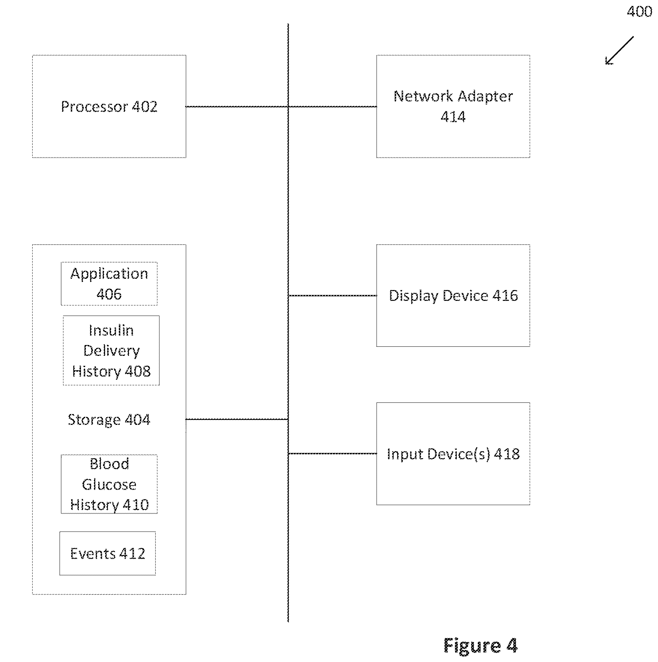

Figure 4 depicts a block diagram of a device 400 suitable for performing the methods that will be described in more detail below. The device 400 includes a processor 402 for executing programming instructions. The processor 402 has access to a storage 404. The storage 404 may store an application 406 for performing the methods. This application 406 may be executed by the processor 402. The storage 404 may store an insulin delivery history 408 for the user. The insulin delivery history 408 may contain data regarding the amount of insulin delivered as well as the date and time of the deliveries. The insulin delivery history 408 may also identify if each delivery is a basal delivery or a bolus delivery. The storage 404 may store the blood glucose history 410. The blood glucose history 410 may include blood glucose concentration level readings as well as the date and time of such readings. These values may be obtained by the glucose monitor 106. The storage 404 additionally may store information regarding events 412, like meal events and exercise events.

The device 400 may include a network adapter 414 for interfacing with networks, like networks 122 and 124. The device 400 may have a display device 416 for displaying video information. The display device 416 may be, for instance, a liquid crystal display (LCD) device, a light emitting diode (LED) device, etc. The device 400 may include one or more input devices 418 for enabling input to be received. Examples of input devices include keyboards, mice, pointing devices, touchscreen displays, button, knobs or the like.

As was mentioned above, the exemplary embodiments may better estimate a user's daily insulin needs. In some conventional insulin delivery systems, the TDI for a user may be estimated based on factors such as body weight, age, lifestyle or other demographics. The TDI may then, in turn, be used to estimate clinical parameters like basal insulin needs, insulin to carbohydrate (I:C) ratios and correction factors. Unfortunately, calculating TDI in such a fashion is error prone and may not accurately reflect a user's daily insulin needs. The exemplary embodiments may seek to overcome this shortcoming of conventional systems by more accurately estimating daily insulin needs of a user. The exemplary embodiments rely upon the actual insulin delivery history of the user to determine the estimate of daily insulin needs.

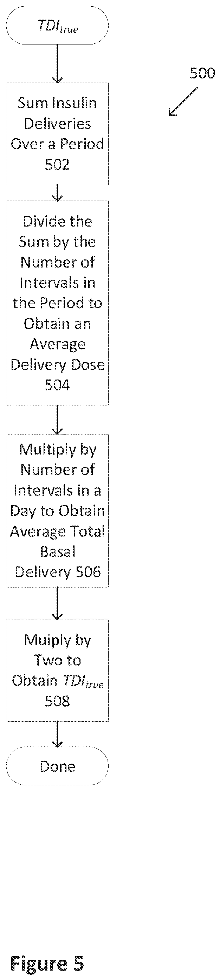

A first aspect of determining the daily insulin needs is to determine a more accurate TDI value than a conventionally calculated TDI. The exemplary embodiments look to the actual automated insulin delivery history to obtain this value. Figure 5 depicts a flowchart of steps (500) that may be performed to obtain the improved TDI value designated as $TDI_{true}$. As a first step, the dosages of automated insulin deliveries to the user from the insulin delivery device 102 over a period of time (L times the length of an interval) are summed (502). This value is divided by $L$ (*i.e.,* the number of intervals in the period of time) to obtain an average insulin delivery for the period (504). The resulting average automated delivery dosage for an interval (such as 5 minutes) is multiplied by the number of intervals in a day to obtain the average total basal insulin delivery per day (506). The number of intervals depends on how often automated insulin delivery is delivered. If the interval is 5 minutes, there are 12 intervals per hour and 24 hours in a day. In such a case, the number of intervals in a day is 12 X 24 or 288. Hence, for that case, the average automated delivery dosage per interval is multiplied by 288 to obtain the total basal insulin delivery per day. The rule of thumb that TDI is 2 times basal may be applied to obtain $TDI_{true}$ by multiplying the value calculated in (506) by two (508).

The resulting equation may be expressed as:

$$TDI_{true} = 2 \frac{\sum_{i=1}^{L} I_{auto}(i)}{L/288}$$

where $I_{auto}(i)$ is the automated insulin delivery dosage at interval $I$.

With the estimate $TDI_{true}$, the exemplary embodiments may estimate accurately an amount of insulin needed to fill a reservoir to match a user's estimated insulin needs over a period, such as three days. This period may be dictated by a length of time that is safe for the reservoir to hold insulin or a predetermined time assumed by insulin delivery device design.

The amount of insulin to be filled into the insulin reservoir may be expressed as:

$$I_{reservoir} = \text{Number } of\ days\ (daily\ basal\ needs + daily\ bolus\ needs).$$

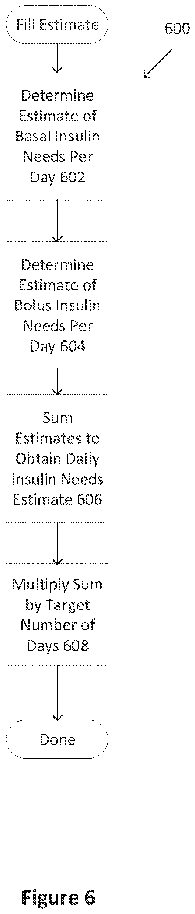

Figure 6 depicts a flowchart 600 depicting steps that may be performed to determine $I_{reservoir}$. First, the daily basal needs of the user are determined (602) by looking to the actual average automated insulin deliveries over a period of time (L intervals) and multiplying the average by the number of intervals in a day. If the interval is 5 minutes, then the average is multiplied by 288. Thus, the daily basal needs may be expressed as:

$$\frac{\sum_{i=1}^{L} I_{auto}(i)}{L/288}$$

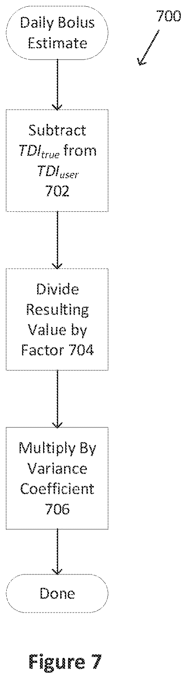

The daily bolus needs may be determined (604). The daily bolus needs may be determined by performing the steps in the flowchart 700 of Figure 7. The process of determining the daily bolus needs begins by calculating the difference between $TDI_{user}$ and $TDI_{true}$(702). $TDI_{user}$ is the average daily sum of all insulin deliveries (basal and bolus) for the user. The difference is divided by a factor, such as 2, (704) because it is assumed that half of TDI is attributable to basal. This factor may, however, be varied based on lifestyle or insulin sensitivity of the user. The resulting value may be multiplied by a variance coefficient (706) to account for variability of boluses, such as a value of 1.2, to obtain the average daily bolus needs. The average daily bolus needs may be expressed as:

$$1.2\frac{TDI_{user} - TDI_{true}}{2}$$

As expressed above, the daily basal needs and the daily bolus needs are summed (606) to obtain the average daily insulin needs. This sum may then be multiplied by the number of days the insulin is intended to last (608), such as was discussed above. If the insulin is intended to last for three days, the sum is multiplied by three.

Hence, $I_{reservoir}$ may be expressed as:

$$I_{reservoir} = 3\left(\frac{\sum_{i=1}^{L} I_{auto}(i)}{L/288} + 1.2\frac{TDI_{user} - TDI_{true}}{2}\right)$$

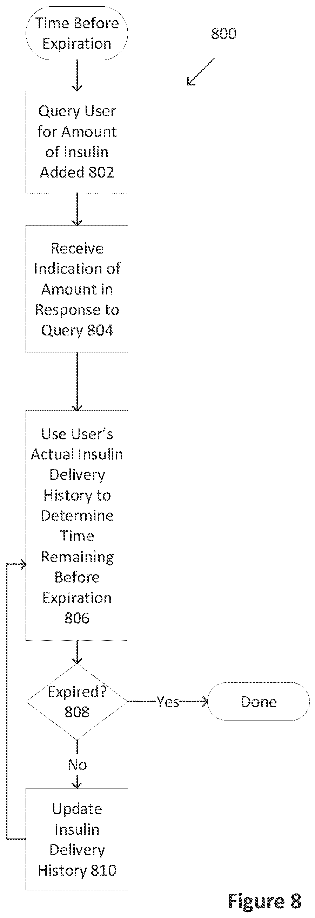

The exemplary embodiments may also estimate how long an amount of insulin in an insulin reservoir will last before being totally consumed by the user. The estimate may be based on the actual insulin use history of the user. Figure 8 depicts a flowchart 800 of steps that may be performed to determine the estimate of how long before the insulin reservoir expires (i.e., will be consumed). At the time of filling the insulin reservoir, the user is queried (such as via a user interface) to provide an amount of insulin in the filled reservoir (802). The amount of insulin is received in response to the query (804). The user's actual insulin delivery history to determine the estimate of time remaining (806). This may be expressed as:

$$\text{Days of insulin remaining} =$$

$$Amount\ filled/\left(\frac{\sum_{i=1}^{L} I_{auto}(i)}{L/288} + 1.2\frac{TDI_{user} - TDI_{true}}{2}\right)$$

In some exemplary embodiments, a check may be made whether the insulin reservoir has expired or not (808). This may entail a simple equation for the amount estimated to be remaining using a variation of the formula

$$Amount\ remaining$$

$$= Amount\ filled$$

$$- Number\ of\ days\ since\ filling\left(\frac{\sum_{i=1}^{L} I_{auto}(i)}{L/288} + 1.2\frac{TDI_{user} - TDI_{true}}{2}\right)$$

Expiration may be reached when the estimated amount remining is below a threshold. Alternatively, a time test could be used. For example, one the determined days of insulin remaining is reached, the reservoir is deemed expired. The value for days remaining may be expressed as a digital value (e.g., 2.7 days) or may be expressed instead in hours or even minutes. If the insulin reservoir has not expired (see 808), the insulin delivery history may be updated each time a delivery is made so that the estimate of insulin remaining is accurate on an ongoing basis (810), and the steps may be repeated after the update beginning at (806) until expiration is reached.

While the present invention has been described with reference to exemplary embodiments described herein, it should be appreciated that various changes in form and detail may be mad without departing from the intended scope of the present invention as defined by the claims appended hereto.

Although the present invention is defined in the attached claims, it should be understood that the present invention can also (alternatively) be defined in accordance with the following embodiments:

1. A method performed by a processor of an electronic device, comprising:

estimating total daily basal insulin needs of a user based on a historic amount of insulin delivered by an automated insulin delivery system to the user per day;

estimating total daily bolus insulin needs of the user based on past insulin bolus deliveries to the user;

adding the estimate of the total daily basal insulin needs of the user with the estimate of the total daily bolus insulin needs of the user to obtain a daily estimate of insulin needs for the user;

determining how many days of insulin for the user are to be stored in a reservoir for the automated insulin delivery system; and

determining that an amount of insulin to be filled in the reservoir is equal to the daily estimate of insulin needs for the user multiplied by the determined number of days of insulin for the user that are to be stored in the reservoir.

2. The method of embodiment 1, wherein the estimating the total daily insulin needs comprises determining an average dosage of insulin delivered by the automated insulin delivery system for a period and multiplying the average dosage by the number of the periods in a day to calculate the amount of insulin delivered by the automated insulin delivery system to the user per day.

3. The method of embodiment 1, wherein the estimating the total daily insulin needs of the user comprises multiplying the amount of insulin delivered by the automated insulin delivery system to the user per day by a factor.

4. The method of embodiment 3, wherein the factor is 2.

5. The method of embodiment 1, further comprising adjusting the estimate of the total daily bolus insulin needs of the user by a safety factor before adding with the estimate of the total daily basal insulin needs of the user.

6. The method of embodiment 1, wherein the estimating total daily bolus insulin needs of the user comprises subtracting two times the estimated total daily basal insulin needs of a user from an average overall sum of insulin deliveries to the user, including basal deliveries and bolus deliveries.

7. The method of embodiment 1, further comprising generating a prompt to the user via a user interface to have the determined amount of insulin to be filled added to the insulin reservoir.

8. A non-transitory computer-readable storage medium storing instruction for execution by a processor to perform the following:

estimate total daily basal insulin needs of a user based on a historical amount of insulin delivered by an automated insulin delivery system to the user per day;

estimate total daily bolus insulin needs of the user based on past insulin bolus deliveries to the user;

add the estimate of the total daily basal insulin needs of the user with the estimate of the total daily bolus insulin needs of the user to obtain a daily estimate of insulin needs for the user;

determine how many days of insulin for the user are to be stored in a reservoir for the automated insulin delivery system; and

determine that an amount of insulin to be filled in the reservoir is equal to the daily estimate of insulin needs for the user multiplied by the determined number of days of insulin for the user that are to be stored in the reservoir.

9. An electronic device, comprising:

a storage for storing a program for determining an amount of insulin to be filled into an insulin reservoir of an insulin delivery system for a user, a history of automated insulin deliveries by the insulin delivery system to the user and a history of bolus insulin deliveries for the user;
a processor for executing the program to perform the following:

estimating total daily basal insulin needs of a user based on a historical amount of insulin delivered by an automated insulin delivery system to the user per day;

estimating total daily bolus insulin needs of the user based on past insulin bolus deliveries to the user;

adding the estimate of the total daily basal insulin needs of the user with the estimate of the total daily bolus insulin needs of the user to obtain a daily estimate of insulin needs for the user;

determining how many days of insulin for the user are to be stored in a reservoir for the automated insulin delivery system; and

determining that an amount of insulin to be filled in the reservoir is equal to the daily estimate of insulin needs for the user multiplied by the determined number of days of insulin for the user that are to be stored in the reservoir.

10. A method performed by a processor of an electronic device, comprising:

when insulin is added to an insulin reservoir of an insulin pump, querying as to how much insulin was added to the reservoir;

receiving in response to the querying a response that specifies an amount of insulin that was added to the reservoir; and

determining when the insulin reservoir will need to be replaced or refilled based on the amount of insulin added to the insulin reservoir and historic data of a user's actual insulin use.

11. The method of embodiment 10, further comprising updating the determining when the insulin reservoir will need to be replaced or refilled based on additional insulin being added to the insulin reservoir and/or historic data of the user's actual insulin use being updated.

12. The method of embodiment 10, wherein the insulin reservoir needs to be replaced or refilled when an amount of insulin in the insulin reservoir is below a threshold.

13. The method of embodiment 10, wherein the insulin reservoir needs to be replaced or refilled when the insulin in the insulin reservoir has been in the reservoir past a time deadline.

14. A non-transitory computer-readable storage medium storing instruction for execution by a processor to perform the following:

when insulin is added to an insulin reservoir of an insulin pump, query as to how much insulin was added to the reservoir;

receive a response to the querying that specifies an amount of insulin that was added to the reservoir; and

determine when the insulin reservoir will need to be replaced or refilled based on the amount of insulin added to the insulin reservoir and historic data of a user's actual insulin use.

15. The non-transitory computer-readable storage medium of embodiment 14, further storing instructions for updating the determining when the insulin reservoir will need to be replaced or refilled based on additional insulin being added to the insulin reservoir and/or updated historic data of the user's actual insulin use.

16. The non-transitory computer-readable storage medium of embodiment 14, wherein the insulin reservoir needs to be replaced or refilled when an amount of insulin in the insulin reservoir is below a threshold.

17. A method performed by a processor of an electronic device, comprising:

receiving an indication of a duration for which an amount of insulin in an insulin reservoir of an insulin pump is to be above a threshold amount;

determining an amount of insulin to be added to the insulin reservoir or replaced in the insulin reservoir in order to keep the amount of insulin in the insulin reservoir above the threshold amount for the specified duration based on historic data of a user's actual insulin use; and

prompting the determined amount of insulin to be added to the reservoir or to be replaced in the insulin reservoir.

18. The method of embodiment 17, wherein the determining the amount of insulin to be added comprises multiplying the average daily insulin use for the user by the duration specified in days.

19. A non-transitory computer-readable storage medium storing instruction for execution by a processor to perform the following:

receive an indication of a duration for which an amount of insulin in an insulin reservoir of an insulin pump is to be above a threshold amount;

determine an amount of insulin to be added to the insulin reservoir or replaced in the insulin reservoir in order to keep the amount of insulin in the insulin reservoir above the threshold amount for the specified duration based on historic data of a user's actual insulin use; and

prompt the determined amount of insulin to be added to the reservoir or to be replaced in the insulin reservoir.

20. The non-transitory computer-readable storage medium of embodiment 19, wherein the determining the amount of insulin to be added comprises multiplying the average daily insulin use for the user by the duration specified in days.

**Claims**

1. A method performed by a processor of an electronic device, comprising:

when insulin is added to an insulin reservoir of an insulin pump, querying as to how much insulin was added to the reservoir;
receiving in response to the querying a response that specifies an amount of insulin that was added to the reservoir; and
determining when the insulin reservoir will need to be replaced or refilled based on the amount of insulin added to the insulin reservoir and historic data of a user's actual insulin use.

2. The method of claim 1, further comprising updating the determining when the insulin reservoir will need to be replaced or refilled based on additional insulin being added to the insulin reservoir and/or historic data of the user's actual insulin use being updated.

3. The method of claim 1, wherein the insulin reservoir needs to be replaced or refilled when an amount of insulin in the insulin reservoir is below a threshold.

4. The method of claim 1, wherein the insulin reservoir needs to be replaced or refilled when the insulin in the insulin reservoir has been in the reservoir past a time deadline.

5. The method of claim 1, wherein the electronic device is one of the insulin pump or a management device for the insulin pump.

6. A non-transitory computer-readable storage medium storing instruction for execution by a processor to perform the following:

   when insulin is added to an insulin reservoir of an insulin pump, query as to how much insulin was added to the reservoir;
   receive a response to the querying that specifies an amount of insulin that was added to the insulin reservoir; and
   determine when the insulin reservoir will need to be replaced or refilled based on the amount of insulin added to the insulin reservoir and historic data of a user's actual insulin use.

7. The non-transitory computer-readable storage medium of claim 6, further storing instructions for updating the determining when the insulin reservoir will need to be replaced or refilled based on additional insulin being added to the insulin reservoir and/or updated historic data of the user's actual insulin use.

8. The non-transitory computer-readable storage medium of claim 6, wherein the insulin reservoir needs to be replaced or refilled when an amount of insulin in the insulin reservoir is below a threshold.

9. The non-transitory computer-readable storage medium of claim 6, wherein the insulin reservoir needs to be replaced or refilled when the insulin in the insulin reservoir has been in the reservoir past a time deadline.

10. The non-transitory computer-readable storage medium of claim 6, further storing instructions for updating an estimate of insulin remaining in the insulin reservoir after each insulin delivery.

11. An electronic device, comprising:

    a storage storing programming code;
    a processor configured to execute the programming code to cause the processor to:

       when insulin is added to an insulin reservoir of an insulin pump, query as to how much insulin was added to the reservoir;
       receive in response to the querying a response that specifies an amount of insulin that was added to the reservoir; and
       determine when the insulin reservoir will need to be replaced or refilled based on the amount of insulin added to the insulin reservoir and historic data of a user's actual insulin use.

12. The electronic device of claim 11, wherein the processor, when executing the programming code, is further configured to: update the determining when the insulin reservoir will need to be replaced or refilled based on additional insulin being added to the insulin reservoir and/or historic data of the user's actual insulin use being updated.

13. The electronic device of claim 11, wherein the insulin reservoir needs to be replaced or refilled when an amount of insulin in the insulin reservoir is below a threshold.

14. The electronic device of claim 11, wherein the insulin reservoir needs to be replaced or refilled when the insulin in the insulin reservoir has been in the reservoir past a time deadline.

15. The electronic device of claim 11, wherein the electronic device is one of the insulin pump or the management device for the insulin pump.

EP 4 693 326 A2

100

Cloud Services/ Server(s) 128

Network 124

Management Device 104
Processor 119
UI 121
Control App. 120
Storage 118
Histories 120

Controller 110
Insulin Reservoir 112

Insulin Delivery Device 102
Histories 113
Storage 114
Control App. 116
User Interface 117

Glucose Monitor 106

User 108

Computing Device 126

Network 122

Figure 1

11

Figure 2

EP 4 693 326 A2

300

Possible Devices for Performing Methods 302

| Computing Device 304 | Management Device 306 | Drug Delivery Device 308 | Cloud Based Service 310 |

**Figure 3**

400

Network Adapter 414

Display Device 416

Input Device(s) 418

Processor 402

Application 406

Insulin Delivery History 408

Storage 404

Blood Glucose History 410

Events 412

Figure 4

$TDI_{true}$

Sum Insulin
Deliveries
Over a Period
502

500

Divide the
Sum by the
Number of
Intervals in
the Period to
Obtain an
Average
Delivery Dose
504

Multiply by
Number of
Intervals in a
Day to Obtain
Average Total
Basal
Delivery 506

Muiply by
Two to
Obtain $TDI_{true}$
508

Done

Figure 5

Fill Estimate

600

Determine Estimate of Basal Insulin Needs Per Day 602

Determine Estimate of Bolus Insulin Needs Per Day 604

Sum Estimates to Obtain Daily Insulin Needs Estimate 606

Multiply Sum by Target Number of Days 608

Done

**Figure 6**

Daily Bolus
Estimate

700

Subtract
$TDI_{true}$ from
$TDI_{user}$
702

Divide
Resulting
Value by
Factor 704

Multiply By
Variance
Coefficient
706

Done

**Figure 7**

```
      ┌─────────────┐
      │ Time Before │                          800
      │ Expiration  │              ↙
      └──────┬──────┘
             │
             ▼
      ┌─────────────┐
      │ Query User  │
      │ for Amount  │
      │ of Insulin  │
      │ Added 802   │
      └──────┬──────┘
             │
             ▼
      ┌─────────────┐
      │   Receive   │
      │ Indication of│
      │  Amount in  │
      │ Response to │
      │  Query 804  │
      └──────┬──────┘
             │
             ▼
      ┌─────────────┐
      │  Use User's │
      │Actual Insulin│
      │   Delivery  │
      │  History to │
      │  Determine  │
      │    Time     │
      │  Remaining  │
      │   Before    │
      │ Expiration  │
      │    806      │
      └──────┬──────┘
             │
             ▼
          ◇ Expired? ◇ ──Yes──▶ ( Done )
          ◇   808   ◇
             │
             No
             │
             ▼
      ┌─────────────┐
      │   Update    │
      │   Insulin   │
      │   Delivery  │
      │ History 810 │
      └─────────────┘
```

Figure 8

**EP 4 693 326 A2**

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 78027820